# EUROPÄISCHE PATENTSCHRIFT

(11) **EP 1 353 684 B1**
(45) Veröffentlichungstag und Bekanntmachung des Hinweises auf die Patenterteilung: **04.05.2011**
(21) Anmeldenummer: 01985432.2
(22) Anmeldetag: 21.12.2001
(51) Int. Cl.: A61K 38/00, A61K 38/03, A61P 9/10, A61P 25/00

(54) **VERWENDUNG VON DIPEPTIDYLPEPTIDASE IV UND AMINOPEPTIDASE-N INHIBITOREN ZUR BEHANDLUNG VON ISCHÄMIE-INDUZIERTER NEURODEGENERATION**
USE OF INHIBITORS OF DIPEPTIDYPEPTIDASE IV (EC 3.3.14.5) AND AMINOPEPTIDASE N (EC 3.4.11.2) FOR THE TREATMENT OF NEURODEGENERATION INDUCED BY ISCHAEMIA
UTILISATION D'INHIBITEURS DE LA DIPEPTIDYLPEPTIDASE IV (EC 3.4.14.5) AINSI QUE DE L'AMINOPEPTIDASE N (EC 3.4.11.2) POUR LE TRAITEMENT DE PROCESSUS NEURODEGENERATIFS D'ORIGINE ISCHEMIQUE

(30) Priorität: 02.01.2001 DE 10100053
(43) Veröffentlichungstag der Anmeldung: 22.10.2003
(73) Patentinhaber: KeyNeurotek Pharmaceuticals AG, 39120 Magdeburg (DE); Ansorge, Siegfried, 39291 Hohenwarte (DE)
(72) Erfinder: ANSORGE, Siegfried, 39291 Hohenwarte (DE); LENDECKEL, Uwe; Inst. Exp. Innere Medizin, 39120 Magdeburg (DE); STRIGGOW, Frank, KeyNeurotek AG i.G., 39120 Magdeburg (DE); NEUBERT, Klaus, Martin-Luther-Uni. Biochemie/Biotech., 06120 Halle (DE); REYMANN, Klaus; c/o F.A.N. GMBH, 39120 Magdeburg (DE); KÄHNE, Thilo, Leipziger Strasse 44, 39120 Magdeburg (DE)
(74) Vertreter: Koepe, Gerd L.
(86) Internationale Anmeldenummer: PCT/EP2001/015196
(87) Internationale Veröffentlichungsnummer: WO 2002/053169

(56) Entgegenhaltungen:
- EP-B- 0 896 538
- EP-B- 1 289 559
- WO-A-98/05333
- WO-A1-01/19866
- WO-A2-2007/127204
- US-A- 5 763 576
- US-A- 2003 148 961
- MARK-KS ET AL.: "Stroke: development, prevention and treatment with peptidase inhibitors" PEPTIDES, Bd. 21, 2000, Seiten 1965-1973, XP002202700
- LOGAN-ME ET AL.: "Bestatin protection against hypoxia induced injury in the guinea-pig heart" JOURNAL OF MOLECULAR AND CELLULAR CARDIOLOGY, Bd. 15, Nr. Suppl.4, 11. Juli 1983 (1983-07-11), Seite 32 XP008004193
- AUGUSTYNS K ET AL: "THE UNIQUE PROPERTIES OF DIPEPTIDYL-PEPTIDASE IV (DPP IV/CD26) AND THE THERAPEUTIC POTENTIAL OF DPP IV INHIBITORS" CURRENT MEDICINAL CHEMISTRY, BENTHAM SCIENCE PUBLISHERS BV, BE, Bd. 6, Nr. 4, 1999, Seiten 311-327, XP000870290 ISSN: 0929-8673
- HALLE J.L.: 'Enzymkinetik: eine Einführung für biochemiker, Mediziner, Biologen, Chemiker und Pharmazeuten', 1987, VEB GUSTAV FISCHER VERLAG
- BARTH A. ET AL: 'Untersuchungen zur Reinigung und Charakterisierung der Dipeptidylaminopeptidase IV' ACTA BIOL MED GER Bd. 32, Nr. 2-3, 1974, Seite 157-174
- SEDO A. ET AL: 'Dipeptidyl peptidase IV-like molecules: homologous proteins or homologous activities?' BIOCHIM BIOPHYS ACTA Bd. 1550, Nr. 2, 17 Dezember 2001, Seite 107-16
- CHEN T. ET AL: 'Dipeptidyl peptidase IV gene family' ADV EXP MED BIOL. Bd. 524, 2003, Seite 79-86
- KAKUTA H. ET AL: 'Fluorescent bioprobes for visualization of puromycin-sensitive aminopeptidase in living cells' BIOORG MED CHEM LETT. Bd. 13, Nr. 1, 06 Januar 2003, Seite 83-86
- EVANS D.M.: 'Dipeptidyl peptidase IV inhibitors.' IDRUGS Bd. 5, Nr. 6, Juni 2002, Seite 577-585
- STÖCKEL-MASCHEK A. ET AL: 'Potent inhibitors of dipeptidyl peptidase IV and their mechanisms of inhibition' ADV EXP BIOL MED Bd. 477, 2000, Seite 117-123
- WENFANG XU ET AL: 'Progress in the development of aminopeptidase N (APN/CD13) inhibitors' CURR MED CHEM - ANTI CANCER AGENTS Bd. 5, 2005, Seite 281-301
- BAUVOIS B. ET AL: 'Aminopeptidase-N/CD13 (EC 3.4.11.2) inhibitors: chemistry, biological evaluations, and therapeutic prospects' MEDICINAL RESEARCH REVIEWS Bd. 26, Nr. 1, 07 Oktober 2005, Seite 88-130
- ALBISTON A.L.: 'Membrane bound members of the M1 family: more than aminopeptidases' PROTEIN AND PEPTIDES LETTERS Bd. 11, Nr. 5, 2004, Seite 491-500
- Biomol- Product Data Sheet Product: Actinonin
- ABBOTT C.A. ET AL: 'Post-proline-cleaving peptidases having DP IV like enzyme activity. Post-proline peptidases' ADV EXP MED BIOL Bd. 477, 2000, Seite 103-109
- DURINX C. ET AL: 'Molecular characterization of dipeptidyl peptidase activity in serum: soluble CD26/dipeptidyl peptidase IV is responsible for the release of X-Pro dipeptides.' EUR J BIOCHEM Bd. 267, Nr. 17, September 2000, Seite 5608-13
- DUKE-COHAN J.S.: 'Attractin: a cub-family protease involved in T cell-monocyte/macrophage interactions.' ADV EXP MED BIOL Bd. 477, 2000, Seite 173-85
- RAWLINGS N.D.; BARRETT A.J.: 'Evolutionary families of metallopeptidases.' METHODS ENZYMOL Bd. 248, 1995, Seite 183-228
- HEO J.H. ET AL: 'Matrix metalloproteinases increase very early during experimental focal cerebral ischemia' J CEREB BLOOD FLOW METAB Bd. 19, Nr. 6, Juni 1999, Seite 624-33
- ROMANIC A.M. ET AL: 'Matrix metalloproteinase expression increases after cerebral focal ischemia in rats: inhibition of matrix metalloproteinase-9 reduces infarct size' STROKE Bd. 29, Nr. 5, Mai 1998, Seite 1020-30
- http://www.sigmaaldrich.com/catalog/search /ProductDetail/SIGMA/B8385 B8385 Bestatin hydrochloride Data-sheet
- http://www.sigmaaldrich.com/catalog/search /ProductDetail/SIGMA/A1276 A1276 Amastatin hydrochloride hydrate - Data-sheet
- http://www.brenda-enzymes.info/ Inhibition profiles for inhibitors: Actinonin, Bestatin, Amastatin

## Beschreibung

Die Erfindung beschreibt die Verminderung Ischämie-bedingter cerebraler Schädigungsprozesses durch die Hemmung der enzymatischen Aktivitäten von Aminopeptidase N (APN, EC3.4.11.2, CD13) und Dipeptidylpeptidase IV (DP IV, EC 3.4.14.5, CD26) oder von Enzymen mit gleicher Substratspezifität (APN-analoger Enzymaktivität bzw. DPIV-analoger Enzymaktivität). Ein neuroprotektiver Effekt wird durch eine kombinierte Applikation jeweils spezifischer Inhibitoren dieser Enzyme auf der Basis von Aminosäurederivaten, Peptiden oder Peptidderivaten erzielt.

Die Druckschrift US-A 5 763 576 offenbart eine Klasse von Tetrapeptid-alpha-Ketoamiden, welche zur selektiven oder generellen Inhibierung von Serin-und Cysteinproteasen verwendet werden kann. Therapeutische Einsatzmöglichkeiten dieser Peptide ist z.B. die Verwendung als Neuroprotektoren bei Ischämie und Schlaganfall.

Die Druckschrift K.S. Mark et al. "Stroke development, prevention and treatment with peptidase inhibitors" in Peptides, Band 21 (2000) 1965 - 1973 offenbart die Verwendung von Peptidase-Inhibitoren, nämlich Inhibitoren des ACE (Angiotensin-converting Enzyme), zur Vorbeugung und Behandlung von Schlaganfall.

Die Druckschrift WO 98/04333 beschreibt beta-Faltblatt-Mimetika als Protease-Inhibitoren, welche sich u.a. zur Behandlung von ischämischen Reperfusions-Schäden eignen.

Die Druckschrift M.E. Logan et al., "Bestatin protection against hypoxia induced injury in the guinea-pig heart" in Journal of molecular and cellular cardiology, Band 15, Ausgabe Nr. 4, 11. Juli 1983, Seite 32 offenbart die Verwendung von Bestatin als potenten Aminpeptidase-Inhibitor zur Verringerung der durch Hypoxie induzierten Störungen in Meerschweinchenherzen.

Die Druckschrift K. Augustyns et al. "The unique properties of dipeptidyl-peptidase IV (DPP IV/CD 26) and the therapeutic potential of DPP IV inhibitors" in Current Medicinal Chemistry, 6 (1999) 311 - 327 ist ein Übersichtsartikel über die Eigenschaften und Funktionen der Dipeptidylpeptidase IV; darüber hinaus werden in dieser Druckschrift Dipeptidylpeptidase IV-Inhibitoren offenbart und deren therapeutisches Potential diskutiert.

Als Ischämie wird die Unterbrechung der Blutversorgung von Zellen, Geweben oder Organen bezeichnet. Diese Situation ist insbesondere dann kritisch, wenn eine kontinuierliche Versorgung mit Sauerstoff und/oder Nährstoffen (z.B. Glucose) notwendig ist. Dies gilt insbesondere für das zentrale Nervensystem (ZNS), da gerade Nervenzellen extrem empfindlich auf eine Unterbrechung der Sauerstoff- und Glucoseversorgung reagieren. Bereits eine kurzzeitige Ischämie, z.B. infolge eines Schlaganfalls oder eines Herzinfarkts, führt zum neuronalen Zelltod in den betroffenen Himarealen. Cerebrale Ischämie ist in den modernen Industriestaaten die häufigste Ursache für Mortalität und Invalidität. So beträgt die jährliche Inzidenzrate des Schlaganfalls in Deutschland ca. 400/100.000 Einwohner.

Die zellulären Mechanismen Ischämie-bedingter Schädigungsprozesse sind vielschichtig und in ihrer Komplexizität bisher nur ungenügend verstanden. Daher sind Maßnahmen zur Prävention und Therapie problematisch [Dirnagl U, Iadecola C, Moskowitz MA (1999) Pathobiology of ischaemic stroke: an integrated view. Trends Neurosci 22:391-39].

Es ist bekannt, daß im Prozess der Aktivierung und klonalen Expansion von Immunzellen, insbesondere von T-Lymphozyten, membranständige Peptidasen wie DP IV oder APN eine Schlüsselrolle spielen [Fleischer B: CD26 a surface protease involved in T-cell activation. Immunology Today 1994; 15:180-184; Lendeckel U et al.: Role of alanyl aminopeptidase in growth and function of human T cells. International Journal of Molecular Medicine 1999; 4:17-27; Riemann D et al.: CD13 - not just a marker in leukemia typing. Immunology Today 1999; 20:83-88]. Verschiedene Funktionen mitogen-stimulierter mononukleärer Zellen (MNZ) oder angereicherter T-Lymphozyten wie DNS-Synthese, Produktion und Sekretion von immunstimulierenden Zytokinen (IL-2, IL-6, IL-12, IFN-γ) und Helferfunktionen für B-Zellen (IgG- und IgM-Synthese) können in Gegenwart von spezifischen Inhibitoren der DP IV und der APN gehemmt werden [Schön E et al.: The dipeptidyl peptidase IV, a membrane enzyme involved in the proliferation of T lymphocytes. Biomed. Biochim. Acta 1985; 2: K9-K15; Schön E et al.: The role of dipeptidyl peptidase IV in human T lymphocyte activation. Inhibitors and antibodies against dipeptidyl peptidase IV suppress lymphocyte proliferation and immunoglobulin synthesis in vitro. Eur. J. Immunol. 1987; 17: 1821-1826; Reinhold D et al.: Inhibitors of dipeptidyl peptidase IV induce secretion of transforming growth factor β1 in PWM-stimulated PBMNC and T cells. Immunology 1997; 91: 354-360; Lendeckel U et al. : Induction of the membrane alanyl aminopeptidase gene and surface expression in human T-cells by mitogenic activation. Biochem. J. 1996; 319: 817-823; Kähne T et al.: Dipeptidyl peptidase IV: A cell surface peptidase involved in regulating T cell growth (Review). Int. J. Mol. Med. 1999; 4: 3-15; Lendeckel U et al.: Role of alanyl aminopeptidase in growth and function of human T cells (Review). Int. J. Mol. Med. 1999; 4: 17-27].

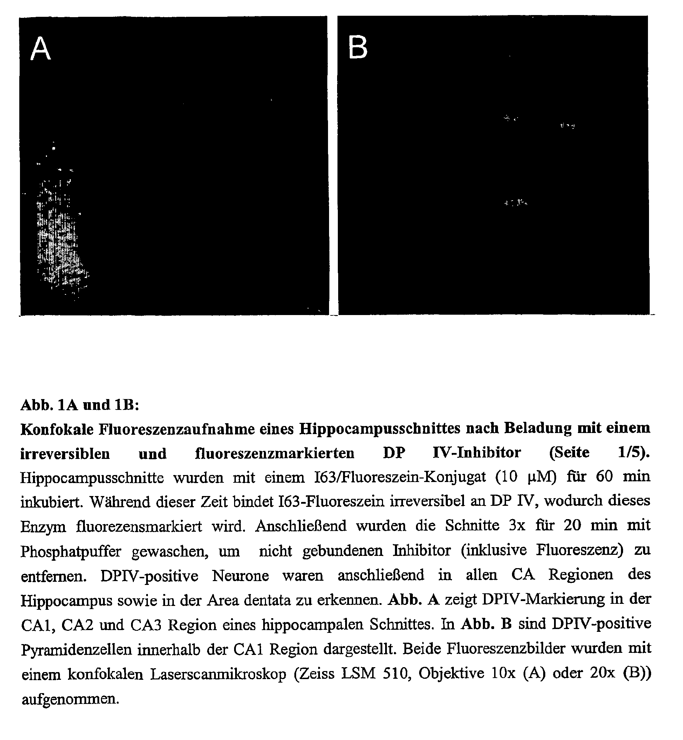

Innerhalb des ZNS sind DP IV und APN auf verschiedenen Zelltypen und Arealen lokalisiert (siehe unten). Kürzlich konnte eine intrazellulär im Cytosol lokalisierte DP IV nachgewiesen werden [Gilmartin L und O'Cuinn G: Neurosci Res 1999; 34: 1-11]. Möglicherweise ist dieses Enzym identisch mit dem von uns erstmals in hippocampalen Pyramidenzellen markierten Protein (Abb. 1, Fluoreszenzaufnahme Hippocampus, I-63/Fluoreszein markiert). Insbesondere diese Nervenzellen sind für ihre herausrausgende Bedeutung für Lern- und Gedächtnisprozesse [Bliss TVP, Collingridge GL (1993) A synaptic model of memory: longterm potentiation in the hippocampus. Nature 361:31-39] sowie für ihre ausgesprochene Sensitivität gegenüber ischämischen Ereignissen bekannt [Striggow F, Riek M, Breder J, Henrich-Noack P, Reymann KG, Reiser G (2000) The protease thrombin is an endogenous mediator of hippocampal neuroprotection against ischemia at low concentrations but causes degeneration at high concentrations. Proc. Natl. Acad. Sci. (USA) 97:2264-2269].

Periphere Immunzellen, beispielsweise T-Zellen, sind normalerweise nicht im ZNS vorhanden. Sie können jedoch in pathologischen Situationen dahin eindringen, insbesondere dann, wenn die Blut/Hirnschranke durchlässig wird. Genau diese Situation ist bei einer cerebralen Ischämie gegeben [Fischer S, Clauss M, Wiesnet M, Renz D, Schaper W, Karliczek GF (1999) Hypoxia induces permeability in brain microvessel endothelial cells via VEGF and NO. Am J Physiol 276:C812-C820]. Die APN ist zudem eine wesentliche Komponente der Zellmembran von Perizyten. Speziell auf Perizyten bestimmen Expression und Aktivität der APN maßgeblich die Funktionalität der Blut-Hirn-Schranke und Beeinträchtigungen der Funktionalität der Blut-Hirn-Schranke gehen mit veränderter Expression der APN einher [Kunz J et al.: J Neurochem. 1994; 62: 2375-2386; Kunz J et al.: J Neuroimmunol. 1995; 59: 41-55; Ramsauer M et al.: J Cereb Blood Flow Metab 1998; 18: 1270-1281; Alliot F et al.: J Neuroscience Research 1999; 58: 367-378].

Sowohl die Dipeptidylpeptidase IV (DPIV) als auch die Alanyl-Aminopeptidase (APN) werden in verschiedenen Arealen des Gehirns exprimiert. Lösliche und Membrangebundene DPIV wurde im Rattenhirn nachgewiesen [Alba F et al.: Peptides 1995; 16: 325-329]. Ebenso konnte DPIV im Zytosol des Meerschweinchenhirns nachgewiesen werden [Gilmartin L und O'Cuinn G: Neurosci Res 1999; 34: 1-11]. Neben dem gut dokumentierten Vorkommen von DPIV auf dem Endothel von Mikrogefäßen des Hirns, wurde das Enzym auf Schwann-ZellMembranen an deren Kontaktstellen zu Axonen sensorischer Nervenendigungen in verschiedenen Säugetieren [Dubovy P und Malinovsky L: Histochemical J 1984; 16: 473-475] sowie an der Luminalmembran von Ependymalzellen in den Ventrikeln und dem zentralen Kanal des Rückenmarks [Boume et al.: Biochem J 1989; 259: 69-80] lokalisiert. DPIV wurde desweiteren eindeutig auf dem Endothel der Mikrovaskulatur des Schweinehirns nachgewiesen sowie dort auch auf Zellen des Striatums [Barnes K et al.: Eur J Neurosci 1994; 6: 531-537].

Die Expression von APN im ZNS erfolgt auf einer breiteren zellulären und räumlichen Basis. Die neuronalen Zelllinien H4 und SK-N-SH, die Oligodendrozyten-Linie MO3.13 sowie die Astrozytenlinien GL-15, U-87 MG und U-373 MG zeichnen sich durch Oberflächenexpression von APN aus [Lachance C et al.: J Virology 1998; 72: 6511-6519]. Diese APN-Oberflächenexpression ist offenbar für den Neurotropismus des humanen Coronavirus 229E verantwortlich [Lachance C et al.: J Virology 1998; 72: 6511-6519]. Neben der Expression auf Gefäßendothel findet sich APN auf Astroglia-Zellen und Perizyten [Barnes K et al.: Eur J Neurosci 1994; 6: 531-537].

Vor dem Hintergrund, daß Inhibitoren der APN und der DPIV nachweislich die Produktion und Freisetzung des immunosuppressiven Zytokins "transforming growth factor-β1" (TGF-β1) aus Immunzellen bewirken [Reinhold D et al.: Immunology 1997; 91: 354-360; Lendeckel U et al. Int J Molecular Medicine 1999; 4: 17-27; Kähne T. et al.: Int J Molecular Medicine 1999; 4: 1-15] ist die Tatsache bemerkenswert, daß dem TGF-β1 offenbar große Bedeutung bei der Begrenzung von Schädigungen des ZNS zukommt. Diese eindeutig neuroprotektiven Funktionen des TGF-β1 konnten bei ischämischen oder exzitotoxischen Läsionen sowie nach Neurotrauma nachgewiesen werden [Ruocco A et al.: Cereb Blood Flow Metab 1999; 19: 1345-1353; Yamashita K et al.: Brain Res 1999; 836: 139-145; Docagne F et al.: FASEB J 1999; 13: 1315-1324; Morganti-Kossmann MC et al.: J Neurotrauma 1999; 16: 617-628; Ho TW et al.: Exp Neurol 2000; 161: 664-675; Henrich-Noack P et al.: Stroke 1996, 27: 1609-1615; Lehrmann E et al.: Exp Neurol 1995; 131: 114-123; Knuckey NW et al.: Brain Res Mol Brain Res 1996; 40: 1-14; Zhu Y et al.: Brain Res 2000; 866: 286-298]. TGF-β1 wird im gesamten Gehirn exprimiert, vergleichsweise höher am Ort von Schädigungen, wobei vorwiegend Astrozyten und Mikroglia-Zellen TGF-β1 produzieren und freisetzen [O'Keefe GM et al.: Eur J Immunol 1999; 29: 1275-1285; Docagne F et al.: FASEB J. 1999; 13: 1315-1324].

Die vorliegende Erfindung betrifft die Verwendung von Inhibitoren der Dipeptidylpeptidase IV (DP IV) oder von Inhibitoren von Enzymen mit gleicher Substratspezifität oder DP IV-analoger Enzymaktivität in Kombination mit Inhibitoren der Alanyl-Aminopeptidase (Aminopeptidase N, APN) oder von Inhibitoren von Enzymen mit gleicher Substratspezifität oder APN-analoger Enzymaktivität zur Herstellung eines Arzneimittels zur Prävention und Therapie Ischämie-induzierter Schädigungen im zentralen Nervensystem, unabhängig davon, ob es sich um akute oder chronische Prozesse und unabhängig davon, ob es sich um Anwendungen am Menschen oder am Tier handelt.

Entsprechend der erfindungsgemäßen Verwendung sind die Inhibitoren der DP IV oder von Enzymen mit gleicher Substratspezifität oder DP IV-analoger Enzymaktivität bevorzugt Xaa-Pro-Dipeptide (Xaa = α-Aminosäure bzw. seitenkettengeschütztes Derivat), vorzugsweise Dipeptidphosphonsäurediarylester, Dipeptidboronsäuren (z.B. Pro-boro-Pro) und deren Salze, Xaa-Xaa-(Trp)-Pro-(Xaa)n-Peptide (Xaa = α-Aminosäure, n = 0 - 10) und deren Salze bzw. Aminosäure (Xaa)-amide und deren Salze, wobei Xaa eine α-Aminosäure bzw. ein seitenkettengeschütztes Derivat, vorzugsweise N^{ε}-4-Nitrobenzyloxycarbonyl-L-Lysin, L-Prolin, L-Tryptophan, L-Isoleucin, L-Valin ist und als Amidstruktur cyclische Amine, z.B. Pyrrolidin, Piperidin, Thiazolidin und deren Derivate fungieren.

Gemäß einer bevorzugten Verwendung werden als Inhibitoren der DP IV oder von Enzymen mit gleicher Substratspezifität oder DP IV-analoger Enzymaktivität Aminosäureamide, z.B. N^{ε}-4-Nitrobenzyloxycarbonyl-L-Lysin-thiazolidid, -pyrrolidid und -piperidid sowie das entsprechende 2-Cyanothiazolidid-, 2-Cyanopyrrolidid- und 2-Cyanopiperidid-Derivat eingesetzt.

Entsprechend der erfindungsgemäßen Verwendung werden als Inhibitoren der APN oder von Enzymen mit gleicher Substratspezifität oder APN-analoger Enzymaktivität bevorzugt Actinonin, Leuhistin, Phebestin, Amastatin, Bestatin (Ubenimex), Probestin, RB3014, β-Aminothiole, α-Aminophosphinsäuren, α-Aminophosphinsäurederivate, vorzugsweise D-Phe-Ψ[PO(OH)-CH₂]-Phe-Phe und deren Salze eingesetzt.

Entsprechend der erfindungsgemäßen Verwendung werden die Inhibitorkombinationen gemäß der vorstehenden Angaben eingesetzt zur Vorbeugung und Therapie von Ischämie-bedingten cerebralen Schädigungen, vorzugsweise nach ischämischem oder hämorrhagischem Schlaganfall, nach Schädel/Him-Trauma, nach Herzstillstand, nach Herzinfarkt oder als Folge von herzchirurgischen Eingriffen (z.B. Bypassoperationen).

Die Inhibitorkombinationen werden simultan mit an sich bekannten Träger-, Hilfs- und/oder Zusatzstoffen verabreicht. Die Verabreichung erfolgt einerseits als systemische Anwendung zur oralen, transdermalen, intravenösen, subcutanen, intracutanen, intramuskulären, rektalen, vaginalen und sublingualen Applikation und andererseits als topische Anwendung in Form von z.B. Cremes, Salben, Pasten, Gelen, Lösungen, Sprays, Liposomen, Schüttelmixturen, Hydrokolloidverbänden bzw. anderen dermatologischen Grundlagen/Vehikeln, einschließlich instillativer Applikation.

Alternativ dazu werden die Inhibitorkombinationen in der Art verwendet, bei der die Inhibitoren der Dipeptidylpeptidase IV (DP IV) oder von Enzymen mit gleicher Substratspezifität oder DP IV-analoger Enzymaktivität und die Inhibitoren der Alanyl-Aminopeptidase (Aminopeptidase N, APN) oder von Enzymen mit gleicher Substratspezifität oder APN-analoger Enzymaktivität in räumlich getrennter Formulierung in Kombination mit an sich bekannten Träger-, Hilfs- und/oder Zusatzstoffen gleichzeitig oder zeitlich unmittelbar aufeinanderfolgend mit dem Ziel einer gemeinsamen Wirkung verabreicht werden.

Der hier beschriebenen Erfindung liegt der überraschende Befund zugrunde, daß die kombinierte Wirkung von Enzyminhibitoren der DP IV (EC 3.4.14.5.) sowie von Enzymen mit gleicher Substratspezifität oder DPIV-analoger Enzymaktivität und der APN (EC3.4.11.2) sowie von Enzymen mit gleicher Substratspezifität oder APN-analoger Enzymaktivität das Überleben neuronaler Zellen in kultivierten Hippocampusschnitten (präpariert aus juvenilen Rattenhirnen) nach Ischämie signifikant verbessert. Dies war bisher nicht bekannt. Unsere Erfindung zeigt daher, daß die kombinierte oder gleichzeitige Verabreichung oben genannter Inhibitoren bzw. entsprechender Zubereitungen und Darreichungsformen daraus zur Prävention und/oder Therapie Ischämie-induzierter cerebraler Schädigungsprozesse geeignet ist.

Bisherige Untersuchungen in unseren Labors zeigten, daß die DNS-Synthese und Proliferation humaner Immunzellen, beispielsweise von peripheren mononukleären Zellen (MNZ) oder T-Lymphozyten durch die simultane und separate Administration von Inhibitoren der DP IV (z.B. Lys[Z(NO₂)]-thiazolidid = I49) und der APN (z.B. Actinonin) gehemmt sowie die Bildung und Sekretion von Zytokinen verändert wird [Reinhold D et al.: Inhibitors of dipeptidyl peptidase IV induce secretion of transforming growth factor β1 in PWM-stimulated PBMNC and T cells. Immunology 1997; 91: 354-360]. Ob ein antiproliferativer Effekt von DP IV- und APN-Inhibitoren auch der hier beschriebenen neuroprotektiven Wirkung beider Substanzklassen zugrunde liegt, verbleibt noch unklar, ist aber wahrscheinlich.

Die Applikation von Enzyminhibitoren der DPIV oder von Enzymen mit gleicher Substratspezifität oder DPIV-analoger Enzymaktivität und der APN oder von Enzymen mit gleicher Substratspezifität oder APN-analoger Enzymaktivität stellt bei den genannten Erkrankungen eine neuartige Methode und ergänzende Therapieform dar. Die erfindungsgemäß applizierten Inhibitoren der DP IV und der APN können in pharmazeutisch anwendbaren Formulierungskomplexen als Inhibitoren, Substrate, Pseudosubstrate, inhibitorisch wirkende Peptide und Peptidderivate sowie als Antikörper dieses Enzyms zur Anwendung kommen. Bevorzugte Effektoren sind beispielsweise für die DP IV Xaa-Pro-Dipeptide vorzugsweise Dipeptidphosphonsäurediarylester und deren pharmazeutisch annehmbaren Salze, Xaa-Xaa-(Trp)-Pro-(Xaa)n-Peptide (n=0-10), und deren pharmazeutisch annehmbaren Salze bzw. Aminosäure (Xaa)-amide, und deren pharmazeutisch annehmbaren Salze, wobei Xaa eine α-Aminosäure/Iminosäure bzw. ein α-Aminosäurederivat/Immosäurederivat, vorzugsweise N^{ε}-4-Nitrobenzyloxycarbonyl-L-Lysin, L-Prolin, L-Tryptophan, L-Isoleucin, L-Valin ist und als Amidstruktur cyclische Amine, z.B. Pyrrolidin, Piperidin, Thiazolidin und deren Derivate. Derartige Verbindungen und deren Herstellung wurden in einem früheren Patent beschrieben (K. Neubert et al. DD296075A5). Bevorzugte Inhibitoren für die Alanyl-Aminopeptidase sind Bestatin (Ubenimex), Actinonin, Probestin, Phebestin, RB3014, Leuhistin, Amastatin, β-Aminothiole, α-Aminophosphinsäuren, α-Aminophosphinsäure-Derivate oder deren pharmazeutisch annehmbaren Salze.

Die Inhibitoren werden simultan oder einzeln mit bekannten Trägerstoffen verabreicht. Die Verabreichung erfolgt einerseits als topische Applikation in Form von z.B. Cremes, Salben, Pasten, Gelen, Lösungen, Sprays, Liposomen, Schüttelmixturen, Hydrokolloidverbänden bzw. anderen dermatologischen Grundlagen/Vehikeln einschließlich instilativer Applikation und andererseits als systemische Applikation zur oralen, transdermalen, intravenösen, subcutanen, intracutanen, intramuskulären rektalen, vaginalen, sublingualen Anwendung in geeigneten Rezepturen bzw. in geeigneter Galenik, zusammen mit an sich bekannten Träger-, ttilfs- und/oder Zusatzstoffen.

### Ausführungsbeispiele

### Beispiel 1

### Neuronale Lokalisation von DP IV (oder eines DP IV-ähnlichen Enzyms) im Hippocampus (siehe Abb. 1, Seite 1/5)

### Beispiel 2

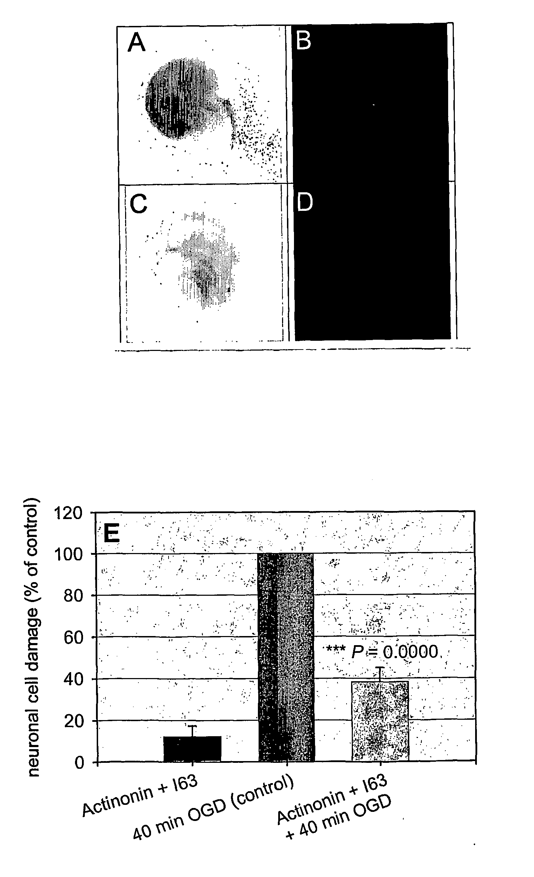

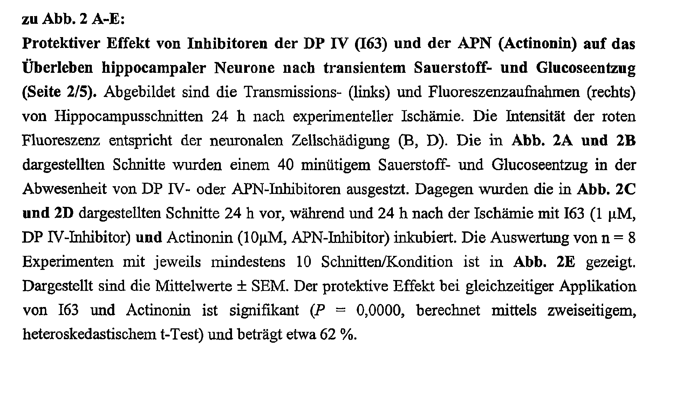

### Neuroprotektive Wirkung gegen experimentelle Ischämie durch Inkubation mit synthetischen Inhibitoren der DP IV und der APN (siehe Abb. 2, Seite 2/5)

Organotypische Hippocampuschnitte wurden entsprechend Striggow F et al. [Striggow F, Riek M, Breder J, Henrich-Noack P, Reymann KG, Reiser G (2000) The protease thrombin is an endogenous mediator of hippocampal neuroprotection against ischemia at low concentrations but causes degeneration at high concentrations. Proc. Natl. Acad. Sci. (USA) 97:2264-2269] präpariert und kultiviert. Nach 12 Tagen in Kultur wurden wurde eine cerebrale Ischämie experimentell durch einen 40 minütigen Sauerstoff- und Glucoseentzug simuliert. Dazu wurde vorübergehend Glucose (10 mM) im Medium durch Mannitol (10 mM) ersetzt sowie mit 95 % N₂ / 5 % CO₂ statt mit 95 % O₂/5 % CO₂ begast. Nach weiteren 24 h unter normalen Kulturbedingungen wurden alle Schnitte für 1h mit Propidiumjodid (PI) inkubiert. PI dringt nur in solche Zellen ein, deren Zellmembran geschädigt ist [Macklis JD, Madison RD (1990) Progressive incorporation ofpropidium iodide in cultured mouse neurons correlates with declining electrophysiological status: a fluorescence scale of membrane integrety. J. Neurosci. Methods 31:43-46]. Erst dann kommt es zur charakteristischen roten Fluoreszenz, deren Intensität sich proportional zur neuronalen Zellschädigung verhält. Die Fluoreszenzbilder wurde mit Hilfe eines inversen Fluoreszenzmikroskopes (Nikon Diaphot, 4x Objektiv) und einer CCD Kamera (Visitron Systems) aufgenommen und anschließend ausgewertet (Nikon, Lucia M Software-Paket).

**Beispiel 3**

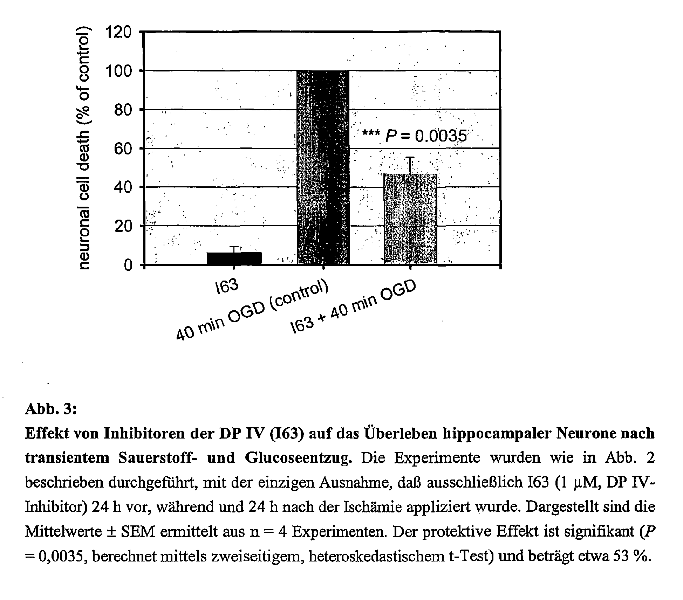

**Protektiver Effekt eines DP IV-Inhibitors in der Abwesenheit von Hemmstoffen der APN** (siehe Abb. 3, Seite 4/5) .

**Beispiel 4**

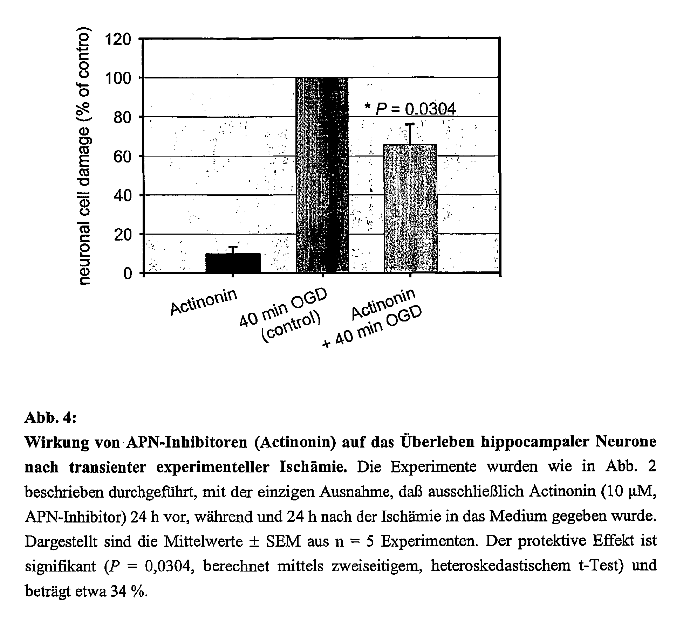

**Protektiver Effekt eines APN-Inhibitors in der Abwesenheit vom Hemmstoffen der DP IV** (siehe Abb. 4, Seite 5/5)

## Patentansprüche

1. Verwendung von Inhibitoren der Dipeptidylpeptidase IV (DP IV) oder von Inhibitoren von Enzymen mit gleicher Substratspezifität oder DP IV-analoger Enzymaktivität in Kombination mit Inhibitoren der Alanyl-Aminopeptidase (Aminopeptidase N; APN) oder von Inhibitoren von Enzymen mit gleicher Substratspezifität oder APN-analoger Enzymaktivität zur Herstellung eines Medikaments zur Prävention und Therapie Ischämie-induzierter Schädigungen im zentralen Nervensystem.

2. Verwendung nach Anspruch 1, worin die Inhibitoren der DP IV ausgewählt sind aus der Gruppe, die besteht aus
- Xaa-Pro-Dipeptiden oder deren pharmazeutisch annehmbaren Salzen, worin Xaa eine α-Aminosäure oder ein seitenkettengeschütztes Derivat ist;
- Xaa-Xaa-(Trp)-Pro-(Xaa)n-Peptiden oder deren pharmazeutisch annehmbaren Salzen,
worin Xaa eine α-Aminosäure ist und n für eine ganze Zahl von 0 bis 10 steht; und
- Aminosäure(Xaa)-amiden oder deren pharmazeutisch annehmbaren Salzen,
worin Xaa eine α-Aminosäure oder ein seitenkettengeschütztes Derivat ist und als Amidstruktur cyclische Amine fungieren.

3. Verwendung nach Anspruch 2, worin das Xaa-Pro-Dipeptid ausgewählt ist aus der Gruppe, die besteht aus Dipeptidphosphonsäurediarylestern, Dipeptidboronsäuren und deren pharmazeutisch annehmbaren Salzen.

4. Verwendung nach Anspruch 3, worin die Dipeptidboronsäure Pro-Boro-Pro ist.

5. Verwendung nach Anspruch 2, worin das Xaa des Aminosäure(Xaa)-amids ausgewählt ist aus der Gruppe, die besteht aus N^{ε}-4-Nitrobenzyloxycarbonyl-L-Lysin, L-Prolin, L-Tryptophan, L-Isoleucin oder L-Valin.

6. Verwendung nach Anspruch 2, worin das cyclische Amin ein Pyrrolidin, ein Piperidin oder ein Thiazolidin ist.

7. Verwendung nach einem der Ansprüche 2 bis 6, worin das Aminosäure(Xaa)-amid ausgewählt ist aus der Gruppe, die besteht aus N^{ε}-4-Nitrobenzyloxy-carbonyl-L-Lysin-thiazolidid, N^{ε}-4-Nitrobenzyloxy-carbonyl-L-Lysin-pyrrolidid, N^{ε}-4-Nitrobenzyloxy-carbonyl-L-Lysin-piperidid, N^{ε}-4-Nitrobenzyloxy-carbonyl-L-Lysin-2-cyanothiazolidid, N^{ε}-4-Nitrobenzyloxycarbonyl-L-Lysin-2-cyanopyrrolidid und N^{ε}-4-Nitrobenzyloxy-carbonyl-L-Lysin-2-cyanopiperidid.

8. Verwendung nach einem der Ansprüche 1 bis 7, worin die Inhibitoren der APN ausgewählt sind aus der Gruppe, die besteht aus Actinonin, Bestatin, Leuhistin, Phebestin, Amastatin, RB3014, Probestin, ß-Aminothiole, α-Aminophosphinsäuren, α-Aminophosphinsäure-Derivaten und deren pharmazeutisch annehmbaren Salzen.

9. Verwendung nach Anspruch 8, worin das α-Aminophosphinsäure-Derivat D-Phe-ψ[PO(OH)-CH₂]-Phe-Phe ist.

10. Verwendung nach einem der Ansprüche 1 bis 9 zur Vorbeugung und Therapie von Ischämie-bedingten cerebralen Schädigungen nach ischämischem oder hämorrhagischem Schlaganfall, nach Schädel/Hirn-Trauma, nach Herzstillstand, nach Herzinfarkt oder als Folge von herzchirurgischen Eingriffen oder von Bypassoperationen.

11. Verwendung nach einem der Ansprüche 1 bis 10 in Kombination mit Träger-, Hilfs- und/oder Zusatzstoffen.

12. Verwendung nach einem der Ansprüche 1 bis 11, worin zwei oder mehrere der Inhibitoren der DP IV bzw. der Inhibitoren von Enzymen mit DP IV-analoger Enzymaktivität und der Inhibitoren der APN bzw. der Inhibitoren von Enzymen mit APN-analoger Enzymaktivität in räumlich getrennter Formulierung in Kombination mit Träger-, Hilfs- und/oder Zusatzstoffen gleichzeitig oder zeitlich unmittelbar aufeinanderfolgend mit dem Ziel einer gemeinsamen Wirkung verabreicht werden.

13. Verwendung nach einem der Ansprüche 1 bis 12 für die systemische Anwendung zur oralen, transdermalen, intravenösen, subcutanen, intracutanen, intramuskulären, rektalen, vaginalen oder sublingualen Applikation in Kombination mit Träger-, Hilfs- und/oder Zusatzstoffen.

14. Verwendung nach einem der Ansprüche 1 bis 12 für die topische Anwendung in Form von Cremes, Salben, Pasten, Gelen, Lösungen, Sprays, Liposomen, Schüttelmixturen, Hydrokolloidverbänden bzw. anderen dermatologischen Grundlagen/Vehikeln, einschließlich instilativer Applikation.

## Claims

1. Use of inhibitors of dipeptidyl peptidase IV (DP IV) or of inhibitors of enzymes having the same substrate specificity or DP IV-analogous enzyme activity in combination with inhibitors of alanyl aminopeptidase (aminopeptidase N, APN) or of inhibitors of enzymes having the same substrate specificity or APN-analogous enzyme activity for the preparation of a medicament for a prevention and therapy of damages in the central nervous system, induced by an ischemia.

2. The use according to claim 1, wherein the inhibitors of DP IV are selected from the group consisting of:
- Xaa-Pro-dipeptides or pharmaceutically acceptable salts thereof, wherein Xaa is an α-amino acid or a side chain protected derivative thereof;
- Xaa-Xaa-(Trp)-Pro-(Xaa)ₙ peptides or pharmaceutically acceptable salts thereof, wherein Xaa is an α-amino acid and n is an integer from 0 to 10; and
- amino acid (Xaa)-amides or pharmaceutically acceptable salts thereof, wherein Xaa is an α-amino acid or a side chain protected derivative thereof and wherein the amide is a cyclic amine.

3. The use according to claim 2, wherein the Xaa-Pro-dipeptide is selected from the group consisting of dipeptide phosphonic acid diaryl esters, dipeptide boronic acids and pharmaceutically acceptable salts thereof.

4. The use according to claim 3, wherein the dipeptide boronic acid is Pro-Boro-Pro.

5. The use according to claim 2, wherein the Xaa of the amino acid (Xaa)-amide is selected from the group consisting of N^{ε}-4-nitrobenzyloxycarbonyl-L-lysine, L-proline, L-tryptophane, L-isoleucine or L-valine.

6. The use according to claim 2, wherein the cyclic amine is a pyrrolidine, a piperidine or a thiazolidine.

7. The use according to any of claims 2 to 6, wherein the amino acid (Xaa)-amide is selected from the group consisting of N^{ε}-4-nitrobenzyloxycarbonyl-L-lysine thiazolidide, N^{ε}-4-nitrobenzyloxycarbonyl-L-lysine pyrrolidide, N^{ε}-4-nitrobenzyloxycarbonyl-L-lysine piperidide, N^{ε}-4-nitrobenzyloxycarbonyl-L-lysine 2-cyanothiazolidide, N^{ε}-4-nitrobenzyloxycarbonyl-L-lysine 2-cyanopyrrolidide and N^{ε}-4-nitrobenzyloxycarbonyl-L-lysine 2-cyano-piperidide.

8. The use according to any of claims 1 to 7, wherein the inhibitors of APN are selected from the group consisting of actinonin, bestatin, leuhistin, phebestin, amastatin, RB3014, probestin, ß-aminothiols, α-aminophosphinic acids, α-aminophosphinic acid derivatives and pharmaceutically acceptable salts thereof.

9. The use according to claim 8, wherein the α-aminophosphinic acid derivative is D-Phe-ψ [PO(OH)-CH₂]-Phe-Phe.

10. The use according to any of claims 1 to 9 for the prevention and therapy of cerebral damages caused by an ischemia after ischemic or hemorrhagic stroke, after a cranial/cerebral trauma, after a heart standstill, after a cardiac infarction or as a consequence of heart surgical operations or of bypass surgeries.

11. The use according to any of claims 1 to 10 in combination with carrier, auxiliary and/or additive substances.

12. The use according to any of claims 1 to 11, wherein two or more inhibitors of the DP IV or the inhibitors of enzymes having DP IV-analogous enzyme activity and the inhibitors of APN or the inhibitors of enzymes having APN-analogous enzyme activity are administered in a compartmentally separate formulation in combination with carrier, auxiliary and/or additive substances simultaneously or immediately consecutively with the aim of a combined effect.

13. The use according to any of claims 1 to 12 for a systemic application for an oral, transdermal, intravenous, subcutaneous, intracutaneous, intramuscular, rectal, vaginal or sublingual administration in combination with carrier, auxiliary and/or additive substances.

14. The use according to any of claims 1 to 12 for a topical application in the form of creams, ointments, pastes, gels, solutions, sprays, liposomes, agitated mixtures, hydrocolloid dressings and other dermatological bases or vehicles, including instillative application.

## Revendications

1. Utilisation d'inhibiteurs de la dipeptidylpeptidase IV (DP IV) ou d'inhibiteurs d'enzymes possédant une spécificité de substrat identique ou une activité enzymatique analogue à celle de la DP IV, en combinaison avec des inhibiteurs de l'alanyl-aminopeptidase (aminopeptidase N ; APN) ou des inhibiteurs d'enzymes possédant une spécificité de substrat identique ou une activité enzymatique analogue à celle de la APN, pour la préparation d'un médicament destiné à la prévention et à la thérapie de dégradations induites par une ischémie dans le système nerveux central.

2. Utilisation selon la revendication 1, dans laquelle les inhibiteurs de la DP IV sont choisis parmi le groupe qui est constitué par :
- des Xaa-Pro-dipeptides ou leurs sels pharmaceutiquement acceptables, Xaa représentant un acide α-aminé ou bien un dérivé dont la chaîne latérale est protégée ;
- des Xaa-Xaa-(Trp)-Pro-(Xaa)ₙ-peptides ou leurs sels pharmaceutiquement acceptables, Xaa représentant un acide α-aminé et n représentant un nombre entier de 0 à 10 ; et
- des (Xaa)-amides d'acides aminés ou leurs sels pharmaceutiquement acceptables, Xaa représentant un acide α-aminé ou bien un dérivé dont la chaîne latérale est protégée, et des amines cycliques faisant office de structure amide.

3. Utilisation selon la revendication 2, dans laquelle le Xaa-Pro-dipeptide est choisi parmi le groupe qui est constitué par des esters diaryliques de l'acide dipeptidephosphonique, des acides dipeptideboriques ainsi que leurs sels pharmaceutiquement acceptables.

4. Utilisation selon la revendication 3, dans laquelle l'acide dipeptideborique est Pro-Boro-Pro.

5. Utilisation selon la revendication 2, dans laquelle le Xaa du (Xaa)-amide d'acide aminé est choisi parmi le groupe qui est constitué par la N^{ε}-4-nitrobenzyloxycarbonyl-L-lysine, -L-proline, -L-tryptophane, -L-isoleucine ou -L-valine.

6. Utilisation selon la revendication 2, dans laquelle l'amine cyclique est une pyrrolidine, une pipéridine ou une thiazolidine.

7. Utilisation selon l'une quelconque des revendications 2 à 6, dans laquelle le (Xaa)-amide d'acide aminé est choisi parmi le groupe qui est constitué par le N^{ε}-4-nitrobenzyloxy-carbonyl-L-lysin-thiazolidide, le N^{ε}-4-nitrobenzyloxy-carbonyl-L-lysin-pyrrolidide, le N^{ε}-4-nitrobenzyloxycarbonyl-L-lysin-pipéridide, le N^{ε}-4-nitrobenzyloxy-carbonyl-L-lysin-2-cyanothiazolidide, le N^{ε}-4-nitrobenzyloxy-carbonyl-L-lysin-2-cyanopyrrolidide et le N^{ε}-4-nitrobenzyloxy-carbonyl-L-lysin-2-cyanopipéridide.

8. Utilisation selon l'une quelconque des revendications 1 à 7, dans laquelle les inhibiteurs de l'APN sont choisis parmi le groupe qui est constitué par l'actinonine, la bestatine, la leuhistine, la phébestine, l'amastatine, le RB3014, la probestine, des β-aminothiols, des acides α-aminophosphiniques, des dérivés de l'acide α-aminophosphinique ainsi que leurs sels pharmaceutiquement acceptables.

9. Utilisation selon la revendication 8, dans laquelle le dérivé de l'acide α-aminophosphinique est le D-Phe-Ψ[PO(OH)-CH₂]-Phe-Phe.

10. Utilisation selon l'une quelconque des revendications 1 à 9, pour la prévention et la thérapie de dégradations cérébrales induites par une ischémie après une attaque d'apoplexie ischémique ou hémorragique, après un traumatisme crânien/cérébral, après un arrêt cardiaque, après un infarctus du myocarde ou suite à des interventions cardiochirurgicales ou à des opérations de pontage.

11. Utilisation selon l'une quelconque des revendications 1 à 10 en combinaison avec des substances de support, des substances auxiliaires et/ou des additifs.

12. Utilisation selon l'une quelconque des revendications 1 à 11, dans laquelle on administre deux des inhibiteurs ou plus de la DP IV respectivement des inhibiteurs d'enzymes possédant une activité enzymatique analogue à celle de la DP IV et des inhibiteurs de la APN respectivement des inhibiteurs d'enzymes possédant une activité enzymatique analogue à celle de la APN dans des formulations séparées dans l'espace en combinaison avec des substances de support, des substances auxiliaires et/ou des additifs, de manière simultanée ou de manière directement successive dans le temps dans le but d'obtenir un effet commun.

13. Utilisation selon l'une quelconque des revendications 1 à 12, pour l'administration systémique à des fins d'application par voie orale, par voie transdermique, par voie intraveineuse, par voie sous-cutanée, par voie intracutanée, par voie intramusculaire, par voie rectale, par voie vaginale ou par voie sublinguale, en combinaison avec des substances de support, des substances auxiliaires et/ou des additifs.

14. Utilisation selon l'une quelconque des revendications 1 à 12, pour l'application locale sous la forme de crèmes, d'onguents, de pâtes, de gels, de solutions, de sprays, de liposomes, de mélanges à agiter, de pansements hydrocolloïdaux respectivement d'autres substrats/véhicules dermatologiques, y compris pour l'application par instillation.
